# FASCICULE DE BREVET EUROPEEN

(11) **EP 0 877 595 B1**
(45) Date de publication et mention de la délivrance du brevet: **06.06.2001**
(21) Numéro de dépôt: 97902420.5
(22) Date de dépôt: 31.01.1997
(51) Int. Cl.: A61K 7/48, A61K 31/11

(54) **COMPOSITION DERMOCOSMETIQUE CONTENANT DU RETINAL**
DERMOKOSMETISCHES MITTEL ENTHALTEND RETINAL
SKIN COSMETIC COMPOSITION CONTAINING RETINAL

(30) Priorité: 02.02.1996 FR 9601276
(43) Date de publication de la demande: 18.11.1998
(73) Titulaire: PIERRE FABRE DERMO-COSMETIQUE, 92100 Boulogne (FR)
(72) Inventeur: NOGUEIRA, Laurent, F-31500 Toulouse (FR); PEYROT, Nicole, F-31400 Toulouse (FR)
(74) Mandataire: Warcoin, Jacques
(86) Numéro de dépôt international: FR9700196
(87) Numéro de publication internationale: WO9727836

(56) Documents cités:
- EP-A- 0 391 033
- WO-A-95/25507
- WO-A-95/26709
- WO-A-96/07396
- DE-A- 4 410 238

## Description

La présente invention concerne des compositions dermocosmétiques contenant du rétinal et un procédé pour améliorer leur stabilité lors de la conservation.

Les produits de soins contenant des rétinoïdes sont devenus un centre d'intérêt ces dernières années. L'acide rétinoïque, connu également sous le nom de Vitamine A acide ou trétinoïne, est utilisé dans le traitement de l'acné et les produits contenant la Vitamine A acide, sont nombreux et variés.

Plus récemment, d'autres applications des rétinoïdes ont été mises en avant, telles que le vieillissement actinique. En effet, les personnes qui se sont beaucoup exposées au soleil pendant leur enfance, présentent, à l'âge adulte, les caractéristiques suivantes: peau burinée, ridée, jaune, relâchée, rugueuse, sèche et présentant une hyperpigmentation, avec souvent apparition de diverses grosseurs malignes. Ces phénomènes sont plus marqués chez les personnes à peau claire qui brûlent souvent et ne bronzent pas.

Un brevet américain U.S. Patent n° 4, 603, 146, décrit le traitement de la peau abîmée par l'exposition solaire, à l'aide d'une préparation contenant de la Vitamine A acide dans un excipient émollient. Plus tard, dans le brevet U.S. Patent n° 4, 877, 805, il est décrit que les rétinoïdes peuvent être utilisés pour prévenir et restaurer les dommages provoqués par le soleil sur la peau humaine.

On sait également que l'utilisation de certains rétinoïdes tels que le rétinal (Vitamine A aldéhyde) (brevets FR 9403339 et FR 9403970) et les esters de Vitamine A (acétate et palmitate) sont préférés à l'acide rétinoïque pour leur meilleure tolérance cutanée. En effet, le rétinal (Vitamine A aldéhyde) par exemple, apparaît naturellement dans le métabolisme humain: il est utilisé dans la vision.

Le brevet FR 2 681 784 a montré l'intérêt du rétinal et de ses précurseurs dans le traitement d'affections telles que la rosacée ou les dermites séborrhéiques.

Toutefois, il s'agit de composés présentant une mauvaise stabilité physico-chimique ; leur formulation sous une forme présentant de bonnes qualités organoleptiques ainsi que de bonnes caractéristiques de conservation n'a pas été résolue de façon satisfaisante à ce jour.

Par exemple, le rétinal actif peut être sous forme 13-cis, 13-trans, ou de leurs mélanges.

Lors de stockage, d'autres formes ont tendance à apparaître, comme le 9-cis-rétinal, le 11-cis-rétinal, ou des produits de condensations de type polymère, qui sont inactives.

Le brevet US 4 826 828 a proposé l'utilisation de silicones volatils et d'éthanol pour la préparation de compositions contenant du rétinol; ces préparations peuvent être diluées avant application par formation d'une émulsion eau/huile.

Le brevet US 4 720 353 décrit des émulsions eau/huile stabilisées par un organopolysiloxane particulier.

Toutefois, ces formulations ne donnent pas des résultats satisfaisants à la conservation.

La demande WO 93/00085 décrit des formulations de rétinoïdes sous forme d'émulsion eau/huile, stabilisées par un système complexe comprenant un agent chélatant et des anti-oxydants hydrosolubles et liposolubles. EP 440 398 concerne également des émulsions eau/huile de rétinoïdes.

Il s'agit de formulations comportant de nombreux paramètres difficiles à mettre en oeuvre. En outre les émulsions eau/huile sont mal adaptées à une application topique, particulièrement en cosmétologie.

Les demandes FR 9403339 et FR 9403970 ont proposé des formulations améliorées de rétinal. Par exemple, la stabilité d'émulsion huile/eau est augmentée, dans FR 9403339, par l'utilisation d'antioxydants liposolubles.

La Demanderesse a maintenant trouvé que la stabilité de compositions à base de rétinal, sous forme d'émulsion huile/eau, pouvait être optimisée par le choix des constituants de la phase huileuse.

C'est pourquoi la présente invention a pour objet une composition dermocosmétique contenant du rétinal, caractérisée en ce qu'il s'agit d'une émulsion huile-dans-eau et en ce que les constituants de la phase grasse ont un indice de peroxyde inférieur ou égal à environ 5.

La stabilisation chimique du rétinal est définie par la concentration persistante de ce dernier sous sa forme chimique originelle et ce, après une durée et une température de stockage définies.

L'élimination de toute matière première présentant un indice de peroxyde supérieur à 5 permet d'obtenir des taux de conservation du rétinal après 12 mois, supérieurs à 97,5% et après 24 mois, supérieurs à 95,5% alors que dans des formulations classiques, il décroît de 35%.

L'indice de peroxyde (Iₚ) est défini dans la Pharmacopée Européenne comme le nombre de milliéquivalents d'oxygène actif contenus dans 1000 g de substance. Il peut être déterminé par des méthodes connues de l'homme du métier.

Des constituants de la phase grasse convenables selon l'invention pourront notamment être choisis dans le groupe des huiles et cires minérales (paraffine, silicone, cire microcristalline), des huiles animales saturées (squalane), des huiles végétales naturelles ou hydrogénées, éthoxylées (huile de ricin hydrogénée éthoxylée), des esters d'acides gras et polyols (stéarate de glycérol) et leurs dérivés éthoxylés (monostéarate de glycérol/POE), les alcools gras naturels ou éthoxylés.

Le rétinal peut être sous forme 13-trans, 13-cis, ou cis-trans et se trouve de préférence dans la phase huileuse de l'émulsion.

Les compositions peuvent contenir en outre au moins un antioxydant, de préférence liposoluble. Il peut être choisi notamment dans le groupe comprenant : le Butyl Hydroxy Toluène (BHT), le Butyl Hydroxy Anisole (BHA), le Palmitate d'ascorbyle, l'Alpha tocophérol et ses esters, l'Acide citrique, le Gallate de Propyle, et/ou leurs mélanges ; la concentration en antioxydant est avantageusement comprise entre 0,005% et 0,5% en poids de la phase huileuse.

Le rétinal est présent de préférence à des concentrations comprises entre 0,01% et 1% en poids de la composition.

Selon l'un des modes de réalisation de l'invention, la phase grasse présente la composition suivante :

| | |
|---|---|
| Paraffine liquide | 5 % |
| Squalane | 3,5% |
| Huile de Ricin hydrogénée éthoxylée | 1 % |
| Monostéarate de glycérol / POE | 8,50 % |
| Cire microcristalline | 1,50% |
| Triglycérides caprique/caprylique | 15 % |
| BHT | 0,02 % |
| Rétinal | 0,05 % |
| 2-Phénoxyéthanol | 0,50 % |
| Parahydroxybenzoate de Propyle | 0,40 % |
| Parahydroxybenzoate de Butyle | 0,20 % |
| Triéthanolamine | 0,30 % qsp pH voisin de 6,5 |

L'invention a également pour objet un procédé de stabilisation d'émulsion huile/eau à base de rétinal, consistant à utiliser des composants présentant un indice de peroxyde inférieur ou égal à 5, à l'exclusion des autres constituants utilisés dans l'art antérieur, tels que les alcools de lanoline acétylés.

Les exemples qui suivent sont destinés à illustrer l'invention sans en limiter la portée.

### Exemple 1

### Etude des indices de peroxyde des matières premières

Dans un premier temps, les indices de peroxyde de différents composés lipophiles ou amphiphiles, ont été déterminés selon la méthode de la Pharmacopée Européenne, IIème Ed., 1980, V, 3-4-5-.

Les composés lipophiles ou amphiphiles testés sont :
- Neobee 18 ® Huile de Carthame hybride
- Neobee M 5 ® Triglycérides caprique et caprylique
- Acetulan ® Alcools de lanoline acétylés
- Solulan PB 10 ® Ether de Lanoline propoxylée
- Cremophor RH 40 ® Huile de ricin hydrogénée éthoxylée
- Simulsol 165 ® Monostéarate de glycérol / POE
- Cire E ® Cire microcristalline
- Cosbiol ® Squalane
- Eumulgin B1 ® Alcool cétyl/stéarylique 12-0E

### Résultats

**Tableau 1**

| Matière première | Indice de peroxyde |
|---|---|
| Neobee 18 ® | 14,81 |
| Huile d'arachide | 13,24 |
| Neobee M5 ® | 0 |
| Acetulan ® | 40,52 |
| Huile d'avoine | 0 |
| Solulan PB 10 ® | 8,09 |
| Emulgin B1 ® | 1,67 |
| Cremophor RH 40 ® | 0 |
| Simulsol 165 ® | 0 |
| Cosbiol ® | 0,29 |
| Cire E ® | 0,50 |

La détermination des indices de peroxyde met en évidence la sensibilité des huiles végétales et des corps gras insaturés et de certaines substances amphiphiles, vis-à-vis de l'oxygène.

### Exemple 2

### Etude de l'amélioration de la stabilisation du Rétinal dans une émulsion huile dans eau

Une étude quantitative de stabilisation du Rétinal dans une émulsion huile dans eau, a été menée sur des formules similaires, qui se différencient entre elles par l'absence de composés lipophiles ou amphiphiles à indice de peroxyde supérieur à 5.

### Formule 1 détaillée

| Phase aqueuse : | | |
|---|---|---|
| Eau purifiée | qsp | 100 % |
| Carbomer | | 0,25 % |
| Propylène glycol | | 3 % |

| Phase grasse : | |
|---|---|
| Huile de Carthame hybride | 10 % |
| Paraffine liquide | 5 % |
| Huile de Ricin hydrogénée éthoxylée | 1 % |
| Monostéarate de glycérol POE | 8,50 % |
| Cire microcristalline | 1,50 % |
| Alcools de Lanoline acétylés | 2 % |
| Triglycérides caprique et caprylique | 10 % |
| Ether de Lanoline propoxylée | 1,50 % |
| BHT | 0,02 % |
| Rétinal | 0,05 % |
| 2-Phénoxyéthanol | 0,50% |
| Parahydroxybenzoate de Propyle | 0,40 % |
| Triéthanolamine | 0,30 % qsp pH voisin de 6,5 |
| Parahydroxybenzoate de Butyle | 0,20 % |

### Formule 2 détaillée

| Phase aqueuse : | | |
|---|---|---|
| Eau purifiée | qsp | 100% |
| Carbomer | | 0,25 % |
| Propylène glycol | | 3% |

| Phase grasse : | |
|---|---|
| Paraffine liquide | 5 % |
| Squalane | 3,5% |
| Huile de Ricin hydrogénée éthoxylée | 1 % |
| Monostéarate de glycérol / POE | 8,50 % |
| Cire microcristalline | 1,50 % |
| Triglycérides caprique et caprylique | 15 % |
| BHT | 0,02 % |
| Rétinal | 0,05 % |
| 2-Phénoxyéthanol | 0,50 % |
| Parahydroxybenzoate de Propyle | 0,40% |
| Parahydroxybenzoate de Butyle | 0,20 % |
| Triéthanolamine | 0,30 % pH voisin de 6,5 |

### Résultats

L'émulsion a été conservée pendant 24 mois à température ambiante (voisine de 20°C)

| Temps de conservation à température ambiante | Pourcentage de trans-rétinal | |
|---|---|---|
| | Formule 1 | Formule 2 |
| Temps 0 | 100% | 100% |
| 6 mois | 95,5 % | 98,5% |
| 12 mois | 80% | 97,7 % |
| 18 mois | 76% | 96,8% |
| 24 mois | 64 % | 95,5% |

La formule 2 présente une bien meilleure stabilité du rétinal que la formule 1, avec :
- 98,5 % de trans-rétinal au bout de 6 mois à température ambiante au lieu de 95,5 %
- 96,8 % au bout de 18 mois à température ambiante au lieu de 76 %,
- et 95,5 % au bout de 24 mois à température ambiante au lieu de 64%.

## Revendications

1. Composition dermocosmétique contenant du rétinal, caractérisée en ce qu'il s'agit d'une émulsion huile-dans-eau et en ce que les constituants de la phase grasse ont un indice de peroxyde inférieur ou égal à environ 5.

2. Composition selon la revendication 1, caractérisée en ce que le rétinal est sous forme, 13-trans 13-cis, ou cis-trans.

3. Composition selon l'une des revendications 1 ou 2, caractérisée en ce que la phase grasse comprend au moins un élément choisi dans le groupe constitué des huiles et cires minérales, des huiles animales saturées, des huiles végétales naturelles ou hydrogénées, éthoxylées, des triglycérides d'acides gras et d'alcool gras, des esters d'acides gras et de polyols, et leurs dérivés éthoxylés, et les alcools gras naturels ou éthoxylés.

4. Composition selon l'une des revendications 1 à 3, caractérisée en ce que la phase grasse comprend au moins un composant choisi dans le groupe comprenant : paraffine, silicone, cire microcristalline, squalane, huile de ricin hydrogénée éthoxylée, stéarate de glycérol, et monostéarate de glycérol/POE.

5. Composition selon l'une des revendications 3 ou 4, caractérisée en ce que la phase grasse contient en outre au moins un antioxydant, à une concentration comprise entre 0,05% et 0,5% en poids de la phase huileuse.

6. Composition selon l'une des revendications 1 à 5, caractérisée en ce que la phase grasse présente la composition suivante :
| | |
|---|---|
| Paraffine liquide | 5% |
| Squalane | 3,5% |
| Huile de Ricin hydrogénée éthoxylée | 1 % |
| Monostéarate de glycérol / POE | 8,50% |
| Cire microcristalline | 1,50% |
| Triglycérides caprique et caprylique | 15 % |
| BHT | 0,02 % |
| Rétinal | 0,05 % |
| 2-Phénoxyéthanol | 0,50 % |
| Parahydroxybenzoate de Propyle | 0,40 % |
| Parahydroxybenzoate de Butyle | 0,20 % |
| Triéthanolamine | 0,30 % |
| | avec un pH voisin de 6,5 |

7. Utilisation de constituants lipophiles présentant un indice de peroxyde inférieur ou égal à 5, pour stabiliser une émulsion huile dans eau contenant du rétinal.

## Claims

1. Dermocosmetic composition containing retinal, characterized in that it is an oil-in-water emulsion and in that the constituents of the fatty phase have a peroxide number of less than or equal to about 5.

2. Composition according to Claim 1, characterized in that the retinal is in 13-trans, 13-cis or cis-trans form.

3. Composition according to either of Claims 1 and 2, characterized in that the fatty phase comprises at least one element chosen from the group consisting of mineral oils and waxes, saturated animal oils, ethoxylated, hydrogenated or natural plant oils, triglycerides of fatty acids and of fatty alcohol, fatty acid esters of polyols, and ethoxylated derivatives thereof, and natural or ethoxylated fatty alcohols.

4. Composition according to one of Claims 1 to 3, characterized in that the fatty phase comprises at least one component chosen from the group comprising: paraffin, silicone, microcrystalline wax, squalane, ethoxylated, hydrogenated castor oil, glyceryl stearate and glyceryl monostearate/POE.

5. Composition according to either of Claims 3 and 4, characterized in that the fatty phase also contains at least one antioxidant, at a concentration of between 0.05% and 0.5% of the weight of the oily phase.

6. Composition according to one of Claims 1 to 5, characterized in that the fatty phase has the following composition:
| | |
|---|---|
| Liquid paraffin | 5% |
| Squalane | 3.5% |
| Ethoxylated hydrogenated castor oil | 1% |
| Glyceryl monostearate/POE | 8.50% |
| Microcrystalline wax - | 1.50% |
| Capric and caprylic triglycerides | 15% |
| BHT | 0.02% |
| Retinal | 0.05% |
| 2-Phenoxyethanol | 0.50% |
| Propyl para-hydroxybenzoate | 0.40% |
| Butyl para-hydroxybenzoate | 0.20% |
| Triethanolamine | 0.30% |
| | with a pH of about 6.5 |

7. Use of lipophilic constituents with a peroxide number of less than or equal to 5, to stabilize an oil-in-water emulsion containing retinal.

## Patentansprüche

1. Dermokosmetische Zusammensetzung, die Retinal enthält, dadurch gekennzeichnet, daß es sich um eine Öl-in-Wasser-Emulsion handelt und daß die Bestandteile der Fettphase eine Peroxidzahl von weniger als oder gleich etwa 5 aufweisen.

2. Zusammensetzung nach Anspruch 1, dadurch gekennzeichnet, daß das Retinal in der Form 13-trans 13-cis oder cis-trans vorliegt.

3. Zusammensetzung nach einem der Ansprüche 1 oder 2, dadurch gekennzeichnet, daß die Fettphase mindestens ein Element enthält, das ausgewählt ist aus der Gruppe bestehend aus Mineralölen und -wachsen, gesättigten tierischen Ölen, natürlichen oder hydrierten, ethoxylierten Pflanzenölen, Triglyceriden von Fettsäuren und Fettalkohol, Ester von Fettsäuren und Polyolen und ihren ethoxylierten Derivaten und natürlichen oder ethoxylierten Fettalkoholen.

4. Zusammensetzung nach einem der Ansprüche 1 bis 3, dadurch gekennzeichnet, daß die Fettphase mindestens einen Bestandteil enthält, der ausgewählt ist aus der Gruppe umfassend: Paraffin, Silicon, mikrokristallines Wachs, Squalan, hydriertes ethoxyliertes Ricinusöl, Gycerinstearat und Monostearat von Glycerin/POE.

5. Zusammensetzung nach einem der Ansprüche 3 oder 4, dadurch gekennzeichnet, daß die Fettphase darüber hinaus ein Antioxidans mit einer Konzentration eingeschlossen zwischen 0,05 und 0,5 Gew.% der Ölphase enthält.

6. Zusammensetzung nach einem der Ansprüche 1 bis 5, dadurch gekennzeichnet, daß die Fettphase die folgende Zusammensetzung aufweist:
| | |
|---|---|
| Flüssiges Paraffin | 5% |
| Squalan | 3,5% |
| Hydriertes ethoxyliertes Ricinusöl | 1% |
| Monostearat von Glycerin/POE | 8,50% |
| Mikrokristallines Wachs | 1,50% |
| Triglyceride von Caprin- und Caprylsäure | 15% |
| BHT | 0,02% |
| Retinal | 0,05% |
| 2-Phenoxyethanol | 0,50% |
| Propylparahydroxybenzoat | 0,40% |
| Butylparahydroxybenzoat | 0,20% |
| Triethanolamin | 0,30% |
| | mit einem pH in der Nähe von 6,5 |

7. Verwendung von lipophilen Bestandteilen, die eine Peroxidzahl von weniger als oder gleich 5 aufweisen, um eine Öl-in-Wasser-Emulsion, die Retinal enthält, zu stabilisieren.
